(19) Europäisches Patentamt
European Patent Office
Office européen des brevets

(11) **EP 4 105 827 B1**

(12) **EUROPEAN PATENT SPECIFICATION**

(45) Date of publication and mention
of the grant of the patent:
**13.03.2024 Bulletin 2024/11**

(21) Application number: **21201801.4**

(22) Date of filing: **11.10.2021**

(51) International Patent Classification (IPC):
**G06V 40/18** *(2022.01)*    **G06V 20/59** *(2022.01)*
**A61B 5/11** *(2006.01)*

(52) Cooperative Patent Classification (CPC):
**G06V 40/197; A61B 5/1103; G06F 18/2178;
G06F 18/28; G06V 20/597; G06V 40/18;
G06V 40/193**

(54) **METHOD AND SYSTEM FOR PERSONALIZED EYE BLINK DETECTION**

VERFAHREN UND SYSTEM ZUR PERSONALISIERTEN ERKENNUNG VON AUGENBLINZELN

PROCÉDÉ ET SYSTÈME DE DÉTECTION PERSONNALISÉE DE BATTEMENTS DES PAUPIÈRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.06.2021 IN 202121026457**

(43) Date of publication of application:
**21.12.2022 Bulletin 2022/51**

(73) Proprietor: **Tata Consultancy Services Limited
Maharashtra (IN)**

(72) Inventors:
• **Gavas, Rahul Dasharath**
**560066 Bangalore, Karnataka (IN)**
• **Karmakar, Somnath**
**700160 Kolkata, West Bengal (IN)**
• **Chatterjee, Debatri**
**700160 Kolkata, West Bengal (IN)**
• **Ramakrishnan, Ramesh Kumar**
**560066 Kolkata, West Bengal (IN)**
• **Pal, Arpan**
**700160 Kolkata, West Bengal (IN)**

(74) Representative: **Goddar, Heinz J.
Boehmert & Boehmert
Anwaltspartnerschaft mbB
Pettenkoferstrasse 22
80336 München (DE)**

(56) References cited:
**EP-A1- 3 056 375**

• **KUMAR ASHISH ET AL: "Driver drowsiness
monitoring system using visual behaviour and
machine learning", 2018 IEEE SYMPOSIUM ON
COMPUTER APPLICATIONS & INDUSTRIAL
ELECTRONICS (ISCAIE), IEEE, 28 April 2018
(2018-04-28), pages 339-344, XP033372027, DOI:
10.1109/ISCAIE.2018.8405495 [retrieved on
2018-07-05]**
• **HOSSAIN MD YOUSUF ET AL: "IOT Based
Real-Time Drowsy Driving Detection System for
the Prevention of Road Accidents", 2018
INTERNATIONAL CONFERENCE ON
INTELLIGENT INFORMATICS AND BIOMEDICAL
SCIENCES (ICIIBMS), IEEE, vol. 3, 21 October
2018 (2018-10-21), pages 190-195, XP033457420,
DOI: 10.1109/ICIIBMS.2018.8550026 [retrieved on
2018-11-27]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

CROSS-REFERENCE TO RELATED APPLICATIONS AND PRIORITY

The present application claims priority from Indian application no. 202121026457, filed on Jun 14, 2021.

TECHNICAL FIELD

The embodiments herein generally relate to automated eye blink detection systems and, more particularly, to a method and system for personalized eye blink detection using passive camera based approach.

BACKGROUND

Eye blink is a bodily function that is indicative of various mental states of a person. Considerable amount of research has been done on various vision-based approaches for detection of eye blinks for cognitive computing. Detection of eye blinks is crucial for applications such as drowsiness detection, for enabling people to better control human computer interfaces, health care applications, to improve ergonomic systems to enable them to prompt users to blink often to avoid dry eyes and computer vision syndrome, monitor cognitive, functions like visual attention and the like. Active approaches for eye blink detection require dedicated special hardware, which are often costly and intrusive in nature, for instance, wearable devices like eyeglasses with special close up cameras observing the eyes, infrared cameras, and illuminators, and so on. On the other hand, passive eye blink detection systems make use of generic devices like web cameras and mobile cameras. The underlying technologies used to detect eye blinks using the above approaches are typically ultrasound technology, electroencephalogram (EEG), infrared light, electromyograph (EMG), electrooculogram (EOG), video signals or the like. However, performance of such approaches is not consistent across participants. Moreover, standard eye tracker or eyeglasses used for detecting blinks, are costly and hence are not suitable for mass deployment.

The detection of eye blinks from a standard video camera makes it possible to use such systems for real time applications owing to their unobtrusiveness and cost effectiveness. There, are several conventional techniques to detect eye blinks from a video sequence. Most of them are based on the estimation of motion in the region around eyes. Such methods first detect the face and eyes using algorithms such as Viola-Jones algorithm or HAAR classifiers. Post face detection, the motion in the eye region is estimated using either sparse tracking or computing the frame-to-frame intensity differences and using thresholding to identify blinks/no blinks. Researchers have also used approaches that detect eye open/closure from single image through techniques like active shape models, using templates of open/close eyes for correlation matching, fitting of models to detect the eye lids. The accuracy of these approaches is significantly affected by conditions like head orientation, resolution of images acquired, motion dynamics, usage of spectacles, face illumination and ambient light. Also, the methods that use raw image intensity are very sensitive to blink estimation. Currently robust facial landmark detectors are available and have been used for eye blink detection, however their performance is not consistent across individuals. Document Kumar Ashish et al., in "Driver drowsiness monitoring system using visual behaviour and machine learning" discloses that: drowsy driving is one of the major causes of road accidents and death. Hence, detection of driver's fatigue and its indication is an active research area. Most of the conventional methods are either vehicle based, or behavioral based or physiological based. Few methods are intrusive and distract the driver, some require expensive sensors and data handling. Therefore, in this paper, a low cost, real time driver's drowsiness detection system is developed with acceptable accuracy. In the developed system, a webcam records the video and driver's face is detected in each frame employing image processing techniques. Facial landmarks on the detected face are pointed and subsequently the eye aspect ratio, mouth opening ratio and nose length ratio are computed and depending on their values, drowsiness is detected based on developed adaptive thresholding. Machine learning algorithms have been implemented as well in an offline manner. A sensitivity of 95.58% and specificity of 100% has been achieved in Support Vector Machine based classification (Abstract).

SUMMARY

Embodiments of the present disclosure present technological improvements as solutions to one or more of the above-mentioned technical problems recognized by the inventors in conventional systems.

For example, in one embodiment, a method for personalized eye blink detection according to claim 1 is provided. The method creates personalized eye blink data for a subject comprising a plurality of images tagged as blink or no-blink, wherein the plurality of images are captured by an image capturing device for one time calibration of a personalized blink threshold for the subject.

The method computes Eye Aspect Ratio (EAR) for each of the plurality of images to obtain a set of EAR values and determines the personalized blink threshold by processing the set of EAR values, wherein the personalized blink threshold comprising one of i) a first blink threshold value, ii) a second blink threshold value, iii) and a pair of blink threshold values, wherein the personalized blink threshold is determined by applying one of: a) a first thresholding approach to compute the first blink threshold value based on a first average and a first standard deviation of each of the set of EAR values computed for each image in a blink tagged image set from among the plurality of images, b) a second thresholding approach to compute the second blink threshold value based on a baseline corrected set of EAR values for the plurality of annotated images utilizing a minimum and a second average of each of the baseline corrected set of EAR values for each image among the plurality of images, and c) a third thresholding approach to compute the pair of blink threshold values based on the second average of each of the baseline corrected set of EAR values for each image from the blink-tagged image set and a no-blink tagged image set among the plurality of images.

The method utilizes the personalized blink threshold to compute a binary decision vector (D) for a plurality of input test images captured over a predefined time window. The value of each element of the binary decision vector (D) computed for each input test image is set to '1' if an EAR value ($y_i$) of corresponding input test image is below the first blink threshold value when the personalized blink threshold value is computed using the first thresholding technique. The value of each element of the binary decision vector (D) computed for each input test image is set to '1' if the baseline corrected EAR value ($yc_i$) of the input test image is below the second blink threshold value when the personalized blink threshold value is computed using the second thresholding technique. The value of each element of the binary decision vector (D) computed for each input test image is set to ' 1' if the difference between the second average for each image from the blink tagged image set and each of the baseline corrected set of EAR values ($yc_i$) is equal or below the difference between the second average for each image from the no-blink tagged image set and each of the baseline corrected set of EAR values, when the personalized blink threshold value is computed using the third thresholding technique. The plurality of input test images are predicted as eye blink if number of elements of the binary decision vector having value '1' are detected for at least the predefined time period.

In another aspect, a system for personalized eye blink detection according to claim 5 is provided. The system comprises a memory storing instructions; one or more Input/Output (I/O) interfaces; and one or more hardware processors coupled to the memory via the one or more I/O interfaces, wherein the one or more hardware processors are configured by the instructions to create personalized eye blink data for a subject comprising a plurality of images tagged as blink or no-blink, wherein the plurality of images are captured by an image capturing device for one time calibration of a personalized blink threshold for the subject.

Further, the system computes Eye Aspect Ratio (EAR) for each of the plurality of images to obtain a set of EAR values and determines the personalized blink threshold by processing the set of EAR values, wherein the personalized blink threshold comprising one of i) a first blink threshold value, ii) a second blink threshold value, iii) and a pair of blink threshold values, wherein the personalized blink threshold is determined by applying one of: a) a first thresholding approach to compute the first blink threshold value based on a first average and a first standard deviation of each of the set of EAR values computed for each image in a blink tagged image set from among the plurality of images, b) a second thresholding approach to compute the second blink threshold value based on a baseline corrected set of EAR values for the plurality of annotated images utilizing a minimum and a second average of each of the baseline corrected set of EAR values for each image among the plurality of images, and c) a third thresholding approach to compute the pair of blink threshold values based on the second average of each of the baseline corrected set of EAR values for each image from the blink-tagged image set and a no-blink tagged image set among the plurality of images.

The system utilizes the personalized blink threshold to compute a binary decision vector (D) for a plurality of input test images captured over a predefined time window. The value of each element of the binary decision vector (D) computed for each input test image is set to '1' if an EAR value ($y_i$) of corresponding input test image is below the first blink threshold value when the personalized blink threshold value is computed using the first thresholding technique. The value of each element of the binary decision vector (D) computed for each input test image is set to '1' if the baseline corrected EAR value ($yc_i$) of the input test image is below the second blink threshold value when the personalized blink threshold value is computed using the second thresholding technique. The value of each element of the binary decision vector (D) computed for each input test image is set to ' 1' if the difference between the second average for each image from the blink tagged image set and each of the baseline corrected set of EAR values ($yc_i$) is equal or below the difference between the second average for each image from the no-blink tagged image set and each of the baseline corrected set of EAR values, when the personalized blink threshold value is computed using the third thresholding technique. The plurality of input test images are predicted as eye blink if number of elements of the binary decision vector having value ' 1' are detected for at least the predefined time period.

In yet another aspect, there are provided one or more non-transitory machine-readable information storage mediums according to claim 9.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 is a functional block diagram of a system, for personalized eye blink detection, in accordance with some embodiments of the present disclosure.
FIG. 2 is a flow diagram illustrating a method for personalized eye blink detection, using the system of FIG. 1, in accordance with some embodiments of the present disclosure.
FIG. 3 illustrates a detailed functional block diagram of the system of FIG. 1, in accordance with some embodiments of the present disclosure.
FIGS. 4A, 4B and 4C depict the various stages in creation of personalized annotated data for personalized eye blink detection by the system of FIG. 1, in accordance with some embodiments of the present disclosure.
FIG. 5 is an eye schematic depicting standard eye landmarks or parameters for eye aspect ratio (EAR) computation.
FIG. 6 depicts raw EAR signal versus a baseline corrected EAR signal.
FIG. 7 depicts performance of system of FIG. 1 as compared to State of Art (SOA) approaches, in accordance with some embodiments of the present disclosure.

It should be appreciated by those skilled in the art that any block diagrams herein represent conceptual views of illustrative systems and devices embodying the principles of the present subject matter. Similarly, it will be appreciated that any flow charts, flow diagrams, and the like represent various processes which may be substantially represented in computer readable medium and so executed by a computer or processor, whether or not such computer or processor is explicitly shown.

DETAILED DESCRIPTION OF EMBODIMENTS

Exemplary embodiments are described with reference to the accompanying drawings. In the figures, the leftmost digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Usage of standard video camera for detection of eye blinks, makes it possible to use such passive systems for real world applications due to their ubiquitous, unobtrusiveness and cost effectiveness. Several techniques are used in literature to detect blinks from a video. However, the accuracy of these approaches is significantly affected by head orientation, resolution of images, motion dynamics, usage of spectacles, face illumination and ambient light. The performance of such existing systems largely depends on accurate identification of eye regions. Due to change in shape and position of eyes, the performance of such systems varies a lot from person to person. Learning the eye blink habits and eye features of an individual is critical to enhance accuracy of eye blink detection/prediction systems when used specifically for monitoring subject of interest.

State of art eye blink detection techniques are generalized systems for usage across individuals, which affects accuracy of eye blink prediction from subject to subject. Embodiments of the present disclosure provide a method and system for personalized eye blink detection using passive camera-based approach. The method first generates a subject specific annotation data, which is then further processed to derive subject specific personalized blink threshold values. The method disclosed provides three unique approaches to compute the personalized blink threshold values which is one time calibration process. The personalized blink threshold is then used to generate a binary decision vector (D) while analyzing a plurality of input test images video sequences) of the subject of interest. Further, values taken by elements of the binary decision vector (D) are analyzed for a predefined time period to predict possible eye blinks of the subject. There are three types of blinks- spontaneous/ natural blinks, reflexive blinks occurring due to external stimulus like air and voluntary blinks resulting from intentional eye closing. The method and system disclosed herein handles detection of spontaneous and voluntary eye blinks under various external conditions by bringing in personalization in video based (passive) blink detection approaches and thereby improving the accuracy of blink detection.

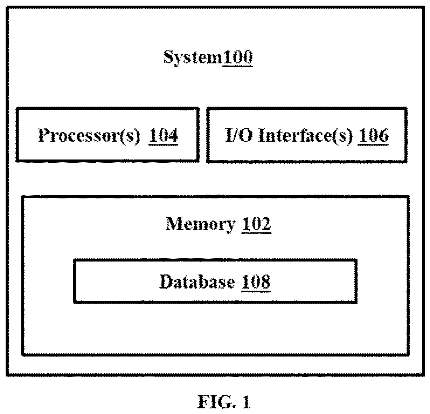

Referring now to the drawings, and more particularly to FIGS. 1 through 7, where similar reference characters denote corresponding features consistently throughout the figures, there are shown preferred embodiments and these embodiments are described in the context of the following exemplary system and/or method.

FIG. 1 is a functional block diagram of a system, for personalized eye blink detection, in accordance with some embodiments of the present disclosure.

In an embodiment, the system 100 includes a processor(s) 104, communication interface device(s), alternatively referred as input/output (I/O) interface(s) 106, and one or more data storage devices or a memory 102 operatively coupled to the processor(s) 104. The system 100 with one or more hardware processors is configured to execute functions of one or more functional blocks of the system 100.

Referring to the components of system 100, in an embodiment, the processor(s) 104, can be one or more hardware processors 104. In an embodiment, the one or more hardware processors 104 can be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102. In an embodiment, the system 100 can be implemented in a variety of computing systems including laptop computers, notebooks, hand-held devices such as mobile phones, workstations, mainframe computers, servers, and the like.

The I/O interface(s) 106 can include a variety of software and hardware interfaces, for example, a web interface, a graphical user interface to display the generated target images and the like and can facilitate multiple communications within a wide variety of networks N/W and protocol types, including wired networks, for example, LAN, cable, etc., and wireless networks, such as WLAN, cellular and the like. In an embodiment, the I/O interface (s) 106 can include one or more ports for connecting to a number of external devices or to another server or devices. External devices, for example, can be an image capturing device such as a camera to receive plurality of images (frames in a video sequence) generated for the calibration phase, generated during stimulated blink tests and natural blink test, and input test images during online eye blink detection phase of the system 100.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes.

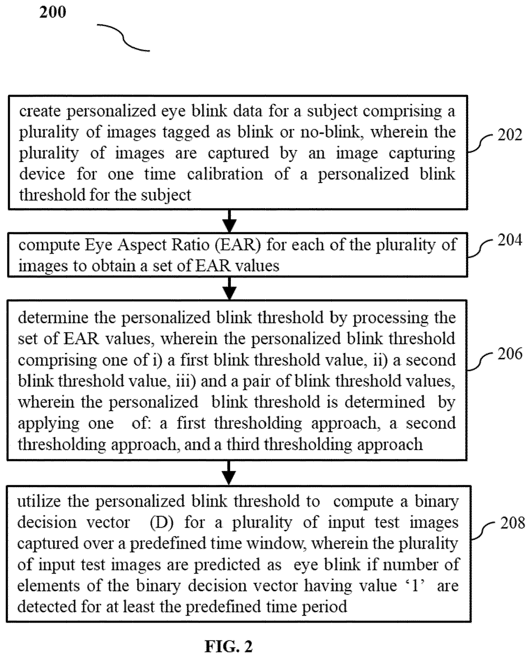

Further, the memory 102 includes a database 108 that stores the collection of the plurality of images, corresponding blink/no-blink tagging generated during calibration phase. The memory 102 also stores determined values of personalized threshold used by the system 100 for personalized eye blink detection, various thresholding techniques as disclosed herein for computing the personalized and so on. Further, the memory 102 may comprise information pertaining to input(s)/output(s) of each step performed by the processor(s) 104 of the system 100 and methods of the present disclosure. In an embodiment, the database 108 may be external (not shown) to the system 100 and coupled to the system via the I/O interface 106. Functions of the components of the system 100 are explained in conjunction with FIG. 2 through FIG. 7.

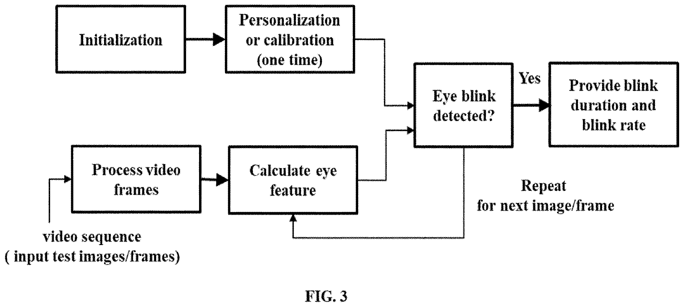

FIG. 2 is a flow diagram illustrating a method 200 for personalized eye blink detection, using the system of FIG. 1, in accordance with some embodiments of the present disclosure. In an embodiment, the system 100 comprises one or more data storage devices or the memory 102 operatively coupled to the processor(s) 104 and is configured to store instructions for execution of steps of the method 200 by the processor(s) or one or more hardware processors 104. The steps of the method 200 of the present disclosure will now be explained with reference to the components or blocks of the system 100 as depicted in FIG. 1 and through FIGS. 3 through 7. Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

Steps of method 200 can be better understood in conjunction with functional block diagram of the system 100, as depicted in FIG. 3, wherein the system 100 functions in two steps, onetime calibration phase to determine personalized blink threshold and a blink detection phase for continuous eyeblink detection based on the computed personalized threshold.

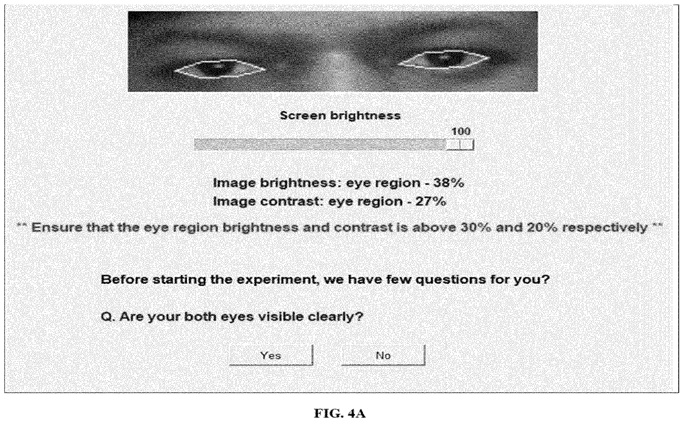

Referring to the steps of the method 200, at step 202 of the method 200, the one or more hardware processors 104 create personalized eye blink data for a subject of interest, hence forth also referred to as subject. The personalized eye blink data comprising a plurality of images, captured via the image capturing device for one time calibration (personalization) of a personalized blink threshold for the subject, wherein the subject is guided to tag (also referred to as annotate) each of the plurality of images as blink or no-blink. For example, the system 100 can be a laptop, and the image capturing device can be the camera of the laptop. As depicted in FIG. 3, the initialization step comprises an application running on the laptop/desktop (with any operating system such as windows™ or Linux™ having an integrated or external webcam. On starting the system 100, the application enables the webcam, and a screen as shown in FIG. 4A appears with the video of user's eye region. It prompts the user to adjust (through a slider) the brightness and contrast levels, so that the eye region is clearly visible. Next, it asks the user whether he is using spectacles and if the ambient light is natural light or artificial light. This data is used only to analyze the system performance under different scenarios.

However, it can be noted that the method 200 can apply one among a plurality of thresholding approaches, wherein in some thresholding approaches do not require the explicit tagging of blink and no-blink data. Thus, in such a scenario, the user is not needed to explicitly perform the annotation, and the annotation block in FIG. 3 is not mandatory to be executed by the system 100.

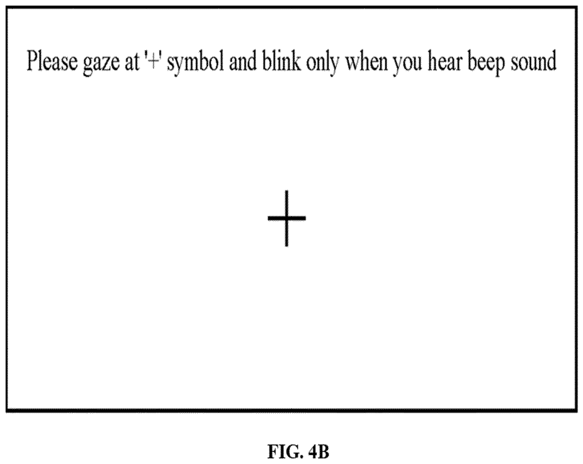

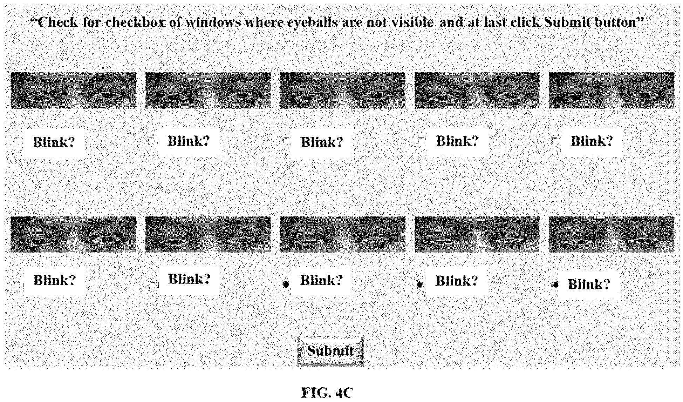

Data collection and annotation for one time calibration: All subjects (participants) for whom the personalized blink thresholds are to be computed are individually requested to face the camera connected to the system 100. The camera captures eye portion video data of the subject and displays on the screen of the system 100, as depicted is FIG. 4A. An application running on the system 100 first provides a setup (using the slider as shown in FIG. 4A) to the subject to properly adjust the brightness and contrast levels, so that the eye region gets detected properly. The video data comprising the plurality of images (video frames) is captured during a stimulus. The stimulus starts with the initial calibration phase with participants fixating at a fixation cross '+' on a blank screen (as depicted in FIG. 4B). A beep sound (2500 Hz of 500ms duration) occurs at different time instances with variable inter beep gaps. The participants are supposed to blink as soon as they hear the beep sound. A total of 5 beeps occur in this phase. An annotation screen as shown in FIG. 4C appears on the screen with each window corresponding to 100 millisecond (ms) of the eye video captured in the calibration phase. The participants are supposed to identify the windows corresponding to the blinks as depicted in FIG. 4C. This annotation is used later for deriving personalized blink threshold computation for each of the participants.

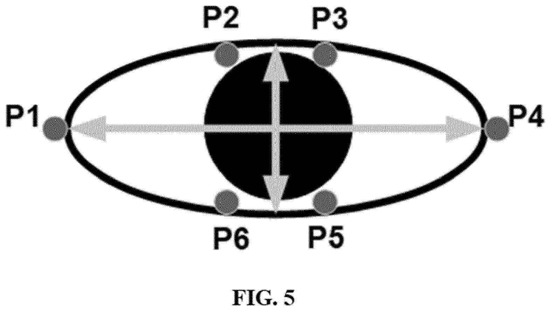

Once all the data collection and/or annotation for the subject of interest is completed, at step 204 of the method 200, the one or more hardware processors 104 compute Eye Aspect Ratio (EAR) for each of the plurality of images to obtain a set of EAR values. The EAR provides the required eye features for personalized eyeblink threshold computation. Standard eye landmarks or parameters, as depicted in FIG. 5, are detected for eyes in every frame or image in the video sequence (plurality of images).. Facial landmark points are extracted using a standard library known in the art such as Dlib™, MediaPipe™ or the like These are used to compute the eye aspect ratio (EAR). The EARe for a given eye (left or right) is computed as,

$$EARe = \frac{||p2 - p6|| + ||p3 - p5||}{2||p1 - p4||} \qquad (1)$$

The final EAR for each image is computed as the average of left and right eyes. The EAR value is high when the eyes are open and decreases when the eyes are closed. As a result, the EAR values corresponding to the blinks are considerably less when compared to the no blink state.

Upon computation of the set of EAR values for each image, at step 206 of the method 200, the one or more hardware processors 104 determine the personalized blink threshold by processing the set of EAR values. The average EAR values ( left eye and right eye) of each image are first interpolated by averaging the neighboring values to get a continuous time series. Continuous EAR time series *x* thus obtained is used for further analysis. The personalized blink threshold is computed via one of the plurality of thresholding techniques disclosed herein. Thus, the personalized blink threshold refers to one of the following threshold values i) a first blink threshold value, ii) a second blink threshold value, iii) and a pair of blink threshold values based on the thresholding technique used by the system 100.

A first thresholding approach, also referred as annotation-based approach (A1): This computes the first blink threshold value based on a first average and a first standard deviation of each of the set of EAR values computed for each image in a blink tagged image set from among the plurality of images. The first thresholding approach is basically a personalization of state of art State of Art (SOA) approaches. The EAR values collected during the calibration phase are segregated as blink tagged image set and a no-blink data tagged image set based upon the annotations provided by the subject during data collection. The first blink threshold value is mathematically expressed as $B_m$ + B$_s$, wherein $B_m$ is the first average of the set of EAR values computed for each image among the blink tagged image set and B$_s$ is the first standard deviation of the set of EAR values computed for each image among the blink tagged image set.

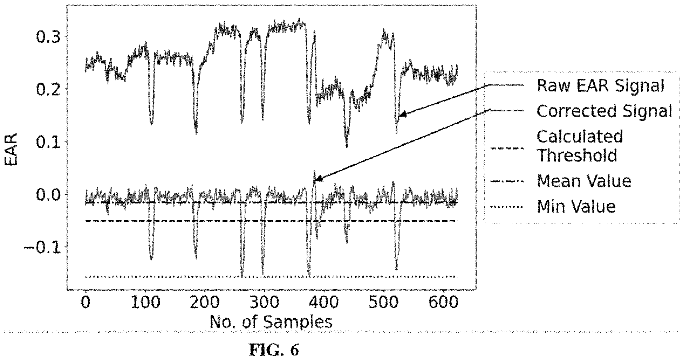

A second thresholding approach also referred as Thresholding based approach (A2): This computes the second blink threshold value based on a baseline corrected set of EAR values for the plurality of annotated images utilizing a minimum and a second average of each of the baseline corrected set of EAR values for each image among the plurality of images. A variety of factors such as lighting conditions, distance from camera, wearing spectacles, and so on, affects the baseline/trend of the EAR signal. This makes the signal non-stationary. Under such circumstances, the first thresholding technique fails considerably. Hence, in the second thresholding technique the EAR series *x* is subjected to baseline correction using the asymmetric least squares smoothing algorithm known in the art. FIG. 6 depicts an example raw EAR signal versus the baseline corrected EAR signal This provides the baseline signal $x_b$, which is then used to obtain the baseline corrected signal, $xc$, where $xc = x - x_b$. The second blink threshold value is mathematically expressed as (t), wherein $t = xc_m - k \times (xc_m - xc_{min})$, wherein $xc_m$ is the second average of each of the baseline corrected set of EAR values for each image among the plurality of images and $xc_{min}$ is the minimum of each of the baseline corrected set of EAR values for each image among the plurality of images, and wherein k is an empirically derived constant.

A third thresholding approach also referred as signal level-based approach (A3): This computes the pair of blink threshold values based on the second average of each of the baseline corrected set of EAR values for each image from the blink-tagged image set and the no-blink tagged image set among the plurality of images. It is possible that when demography changes, the constant *k* as derived in second thresholding approach can vary affecting the results. Hence, we have devised a method which is independent of any constant values. Thus, in the third thresholding approach the average values of blink and no-blink portions of *xc* are computed as, $xc_{Mb}$ and $xc_{Mnb}$, respectively. These two values are stored as a personalization parameters and are also referred as pair of threshold values. The $xc_{Mb}$ and $xc_{Mnb}$ are parameters of a conditional mathematical equation expressed as $|xc_{Mb} - yc_i| \le |xc_{Mnb} - yc_i|$. Thus, $xc_{Mb}$ is the second average of each of the base line corrected set of EAR values for each image from the blink tagged image set, and $xc_{Mnb}$ is the second average of each of the base line corrected set of EAR values for each image from the no-blink tagged image set. Thus, the conditional mathematical equation is independent of a constant '*k*'.

Once the personalized threshold value is computed, then at step 208 of the method 200, the one or more hardware processors 104 utilize the personalized blink threshold value to compute a binary decision vector (D) for the plurality of input test images captured over a predefined time window. The plurality of input test images may be captured offline or in other implementation may be captured online, based on requirements of the end application of the system 100. The value of each of the element of the binary decision vector is computed in accordance with the thresholding approach that is been applied during calibration phase.

For the first thresholding approach, value of each element of the binary decision vector (D) computed for each input test image is set to '1' if the EAR value ($y_i$) of corresponding input test image is below the first blink threshold value. The decision vector (D) for the first approach is mathematically computed as in equation 2 below:

$$D = \begin{cases} 1, if\ y_i\ <\ (B_m\ +\ B_s\,) \\ 0, otherwise \end{cases} \qquad (2)$$

V,i = 1,2,...,N number of EAR values in the data associated with input test images. Instances of '1's in D corresponding to at least 100ms are considered as blinks. The value 100ms, that defines a possible blink is determined empirically.

For the second thresholding approach, value of each element of the binary decision vector (D) computed for each input test image is set to ' 1' if the baseline corrected EAR value ($yc_i$) of the input test image is below the second blink threshold value. The example raw EAR signal and corresponding baseline corrected EAR signal is depicted in FIG. 6. The decision vector (D) for the second approach is mathematically computed as in equation 3 below:

$$D = \begin{cases} 1, yc_i < t \\ 0, if\ otherwise \end{cases} \qquad (3)$$

V,i = 1,2,...,N number of EAR values in the data associated with input test images. Instances of '1's in D corresponding to at least 100ms are considered as blinks. Wherein, $t = xc_m - k \times (xc_m - xc_{min})$ and the value 100ms, that defines the possible blink is determined empirically.

For the third thresholding approach the value of each element of the binary decision vector (D) computed for each input test image is set to '1' if the - difference between the second average for each image from the blink tagged image set and each of the baseline corrected set of EAR values ($yc_i$) is below the difference between the second average for each image from the no-blink tagged image set and each of the baseline corrected set of EAR values. The decision vector (D) for the third approach is mathematically computed as in equation 4 below:

$$D = \begin{cases} 1,\ if\ |xc_{Mb} - yc_i| \le |xc_{Mnb} - yc_i| \\ 0, if\ otherwise \end{cases} \qquad (4)$$

V,i = 1,2,...,N number of EAR values in the data associated with input test images. Instances of '1's in D corresponding to at least 100ms are considered as blinks. The value 100ms, that defines the possible blink is determined empirically.

Once the eye blinks are detected, this data can be forwarded to any end application to derive insights from the eye blink data of the subject. The examples of end application can be drowsiness detection, liveliness monitoring and the like.

Also, stimulus for validation of the system 100 is collected, where two types of eyeblink data are collected for validation using a stimulated blink test and a natural blink test.

Stimulated blink test: The stimulus in this case is same as that of the calibration phase with the participants supposed to blink after the beep sounds. A total of 6 beeps with different inter beep sounds is used.

Natural blink test: Since, stimulated blinks have somewhat known definite patterns in terms of occurrence, duration and intensity, natural blinks are also considered. In this phase, the subjects are allowed to blink naturally and then enter the number of blinks in the provided application. Three such trials are conducted.

EXPERIMENTAL RESULTS: The performance evaluation is done for stimulated and natural blinks. The videos (plurality input test images) are processed frame by frame. The EAR time series is constructed for each frame and baseline correction is performed. Next, these EAR values are compared with user specific thresholds (computed personalization thresholds) and a decision (blink or no-blink) is taken. For each detected blink, corresponding blink amplitude and duration is also reported.

The performance of the system 100 for two types of light, Natural Light (NL) and Artificial Light (AL), two types of blinks (stimulated and natural) and usage of spectacles (WS and WoS) were analyzed using Fscore, which is the harmonic mean of precision and recall. Average Fscores across participants for individual scenarios are presented in TABLE I.

TABLE I

| Methods | With spec | W/o spec | Artif. light | Nat. light | Simu. blink | Nat. blink |
|---|---|---|---|---|---|---|
| **Annotation based (A1)** | 0.81 (0.26) | 0.89 (0.19) | 0.85 (0.22) | 0.87 (0.22) | 0.86 (0.2) | 0.85 (0.26) |
| **Threshold based (A2)** | **0.97 (0.07)** | **0.98 (0.03)** | **0.98 (0.04)** | **0.98 (0.05)** | **0.94 (0.18)** | **0.97 (0.1)** |
| **Signal level based (A3)** | **0.98 (0.05)** | **0.99 (0.03)** | **0.98 (0.04)** | **0.98 (0.04)** | **0.95 (0.18)** | **0.98 (0.11)** |
| **SOA1** | 0.88 (0.15) | 0.9 (0.15) | 0.91 (0.14) | 0.87 (0.17) | 0.83 (0.24) | 0.9 (0.15) |
| **SOA2** | 0.48 (0.37) | 0.37 (0.38) | 0.41 (0.4) | 0.40 (0.37) | 0.28 (0.28) | 0.45 (0.4) |
| **SOA3** | 0.69 (0.36) | 0.7 (0.33) | 0.74 (0.34) | 0.67 (0.34) | 0.71 (0.34) | 0.68 (0.35) |

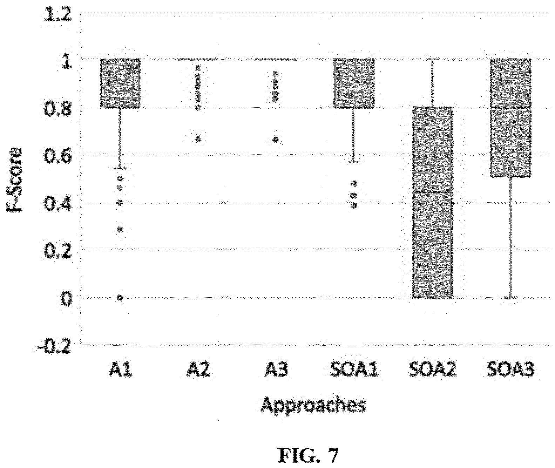

It is observed that threshold-based and signal level-based approaches have similar performance and give the maximum Fscore across different models for all scenarios. The method disclosed herein is also compared with few state of the art (SOA) approaches. Huda et al. ("Mobile-based driver sleepiness detection using facial landmarks and analysis of EAR values", SOA1) and Mallikarjuna ("Liveness detection based on human eye blinking for photo attacks", SOA2) have used fixed threshold values of 0.24 and 0.3 respectively, whereas A. Mohammed used HAAR classifier ("Efficient eye blink detection method for disabled-helping domain", SOA3) based approaches. These SOA approaches are applied to collected datasets and the average Fscores across participants are presented in TABLE I. The Fscores for various scenarios across participants using the three thresholding approaches (A1, A2, A3) of the system 100 and state of the art approaches are shown in FIG. 7. It is seen that A1 is performing similar to SOA1, however, A2 and A3 outperforms all SOA approaches for all scenarios. Hence it indicates that method disclosed herein performs significantly better than the SOAs. Finally, the performance of (A1, A2, A3) on publicly available eyeblink8 dataset is analyzed. SOA1, SOA2 and SOA3 have also used this dataset. The dataset contains blink data from 8 participants and annotations as ground truth. The results obtained using (A1, A2, A3) approaches are given in TABLE II.

TABLE II

| Approach | A1 | A2 | A3 | SOA1 | SOA2 | SOA3 |
|---|---|---|---|---|---|---|
| **Average** | 0.55 | **0.91** | 0.86 | 0.62 | 0.25 | 0.21 |
| **SD** | 0.26 | **0.06** | 0.11 | 0.25 | 0.27 | 0.26 |

Three participants have recordings of duration less than 3 minutes (minimum is 2 min 40 sec.). Goal was to find a solution that performs equally well across different users. The data set has varying sizes of data for different users ranging from 2.5 minutes to 9 minutes and hence to bring uniformity across participants, we calculated the results on a frame of duration 2 min 30 seconds. The first 60 seconds of data was used to personalize the system 100 for the user. It is observed, that both A2 and A3 outperform SOA approaches for this dataset also, however, since A3 relies on self-annotation, performance is not as good as A2. Thus, A2 is more robust compared to A3.

The method and system disclosed herein can be implemented in any computers having an internal or external webcam. Thus, the system is easy to deploy as it does not require any specialized or expensive hardware. Further, the system is ubiquitous and unobtrusive in nature and addresses the technical problem of introducing personalization in blink detection, which provides consistent accuracy of blink detection even when used across different users.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a scope of disclosed embodiments being indicated by the following claims.

**Claims**

1. A processor implemented method (200) for personalized eye blink detection, the method comprises:

   creating (202), by one or more hardware processors, personalized eye blink data for a subject comprising a plurality of images tagged as blink or no-blink, wherein the plurality of images are captured by an image capturing device for capturing eye region video data of the subject for one time calibration of a personalized blink threshold for the subject, wherein a setup is provided to adjusts a brightness and contrast levels by the subject, and wherein the setup guides the subject to tag each of the plurality of images as blink or no-blink;
   computing (204), by the one or more hardware processors, an Eye Aspect Ratio (EAR) for each of the plurality of images to obtain a set of EAR values;
   determining (206), by the one or more hardware processors, the personalized blink threshold by processing the set of EAR values, wherein the personalized blink threshold comprising one of i) a first blink threshold value, ii) a second blink threshold value, iii) and a pair of blink threshold values, wherein the personalized blink threshold is determined by applying one of:

a) a first thresholding approach to compute the first blink threshold value based on a first average and a first standard deviation of each of the set of EAR values computed for each image in a blink tagged image set from among the plurality of images,

b) a second thresholding approach to compute the second blink threshold value based on a baseline corrected set of EAR values for the plurality of annotated images utilizing a minimum and a second average of each of the baseline corrected set of EAR values for each image among the plurality of images, and

c) a third thresholding approach to compute the pair of blink threshold values based on the second average of each of the baseline corrected set of EAR values for each image from the blink-tagged image set and a no-blink tagged image set among the plurality of images;

capturing a plurality of input test images, by the one or more hardware processors, over a predefined time window; and

utilizing (208), by the one or more hardware processors, the personalized blink threshold to compute a binary decision vector (D) for the plurality of input test images,

wherein the value of an element of the binary decision vector (D) computed for a corresponding input test image is set to '1' if an EAR value ($y_i$) of the input test image is below the first blink threshold value when the personalized blink threshold value is computed using the first thresholding technique,

wherein the value of an element of the binary decision vector (D) computed for a corresponding input test image is set to '1' if the baseline corrected EAR value ($yc_i$) of the input test image is below the second blink threshold value when the personalized blink threshold value is computed using the second thresholding technique,

wherein the value of an element of the binary decision vector (D) computed for a corresponding input test image is set to '1' if the difference between the second average for each image from the blink tagged image set and each of the baseline corrected set of EAR values ($yc_i$) is equal or below the difference between the second average for each image from the no-blink tagged image set and each of the baseline corrected set of EAR values, when the personalized blink threshold value is computed using the third thresholding technique, and

wherein the plurality of input test images are predicted as eye blink if a defined number of elements of the binary decision vector have the value '1' are detected for at least the predefined time period.

2. The method as claimed in claim 1, wherein the first blink threshold value is mathematically expressed as $B_m + B_s$, wherein $B_m$ is the first average of the set of EAR values computed for each image among the blink tagged image set and $B_s$ is the first standard deviation of the set of EAR values computed for each image among the blink tagged image set.

3. The method as claimed in claim 1, wherein the second blink threshold value mathematically expressed as (t), wherein $t = xc_m - k \times (xc_m - xc_{min})$, wherein $xc_m$ is the second average of each of the baseline corrected set of EAR values for each image among the plurality of images and $xc_{min}$ is the minimum of each of the baseline corrected set of EAR values for each image among the plurality of images, and wherein k is an empirically derived constant.

4. The method as claimed in claim 1, wherein the pair of threshold values is parameter of a conditional mathematical equation expressed as $|xc_{Mb} - yc_i| \leq |xc_{Mnb} - yc_i|$, wherein $xc_{Mb}$ is the second average of each of the base line corrected set of EAR values for each image from the blink tagged image set, and $xc_{Mnb}$ is the second average of each of the base line corrected set of EAR values for each image from the no-blink tagged image set, and wherein the conditional mathematical equation is independent of a constant 'k'.

5. A system (100) for personalized eye blink detection, the system (100) comprising:

a memory (102) storing instructions;
one or more Input/Output (I/O) interfaces (106); and
one or more hardware processors (104) coupled to the memory (102) via the one or more I/O interfaces (106), wherein the one or more hardware processors (104) are configured by the instructions to:

create personalized eye blink data for a subject of interest comprising a plurality of images tagged as blink or no-blink, wherein the plurality of images are captured by an image capturing device for capturing eye region video data of the subject for one time calibration of a personalized blink threshold for the subject, wherein a setup is provided to adjusts a brightness and contrast levels by the subject, and wherein the setup guides the subject to tag each of the plurality of images as blink or no-blink;

compute Eye Aspect Ratio (EAR) for each of the plurality of images to obtain a set of EAR values;
determine the personalized blink threshold by processing the set of EAR values, wherein the personalized blink threshold comprising one of i) a first blink threshold value, ii) a second blink threshold value, iii) and a pair of blink threshold values, wherein the personalized blink threshold is determined by applying one of:

a) a first thresholding approach to compute the first blink threshold value based on a first average and a first standard deviation of each of the set of EAR values computed for each image in a blink tagged image set from among the plurality of images,
b) a second thresholding approach to compute the second blink threshold value based on a baseline corrected set of EAR values for the plurality of annotated images utilizing a minimum and a second average of each of the baseline corrected set of EAR values for each image among the plurality of images, and
c) a third thresholding approach to compute the pair of blink threshold values based on the second average of each of the baseline corrected set of EAR values for each image from the blink-tagged image set and a no-blink tagged image set among the plurality of images;

capture a plurality of input test images over a predefined time window;
utilize the personalized blink threshold to compute a binary decision vector (D) for the plurality of input test images,
wherein the value of an element of the binary decision vector (D) computed for a corresponding input test image is set to '1' if an EAR value ($y_i$) of the input test image is below the first blink threshold value when the personalized blink threshold value is computed using the first thresholding technique,
wherein the value of an element of the binary decision vector (D) computed for a corresponding input test image is set to '1' if the baseline corrected EAR value ($yc_i$) of the input test image is below the second blink threshold value when the personalized blink threshold value is computed using the second thresholding technique,
wherein the value of an element of the binary decision vector (D) computed for a corresponding input test image is set to '1' if the difference between the second average for each image from the blink tagged image set and each of the baseline corrected set of EAR values ($yc_i$) is equal or below the difference between the second average for each image from the no-blink tagged image set and each of the baseline corrected set of EAR values, when the personalized blink threshold value is computed using the third thresholding technique, and
wherein the plurality of input test images are predicted as eye blink if a defined number of elements of the binary decision vector have the value '1' are detected for at least the predefined time period.

6. The system as claimed in claim 5, wherein the first blink threshold value is mathematically expressed as $B_m + B_s$, wherein $B_m$ is the first average of the set of EAR values computed for each image among the blink tagged image set and $B_s$ is the first standard deviation of the set of EAR values computed for each image among the blink tagged image set.

7. The system as claimed in claim 5, wherein the second blink threshold value mathematically expressed as (t), wherein $t = xc_m - k \times (xc_m - xc_{min})$, wherein $xc_m$ is the second average of each of the baseline corrected set of EAR values for each image among the plurality of images and $xc_{min}$ is the minimum of each of the baseline corrected set of EAR values for each image among the plurality of images, and wherein k is an empirically derived constant.

8. The system as claimed in claim 5, wherein the pair of threshold values are parameters of a conditional mathematical equation expressed as $|xc_{Mb} - yc_i| \leq |xc_{Mnb} - yc_i|$, wherein $xc_{Mb}$ is the second average of each of the base line corrected set of EAR values for each image from the blink tagged image set, and $xc_{Mnb}$ is the second average of each of the base line corrected set of EAR values for each image from the no-blink tagged image set, and wherein the conditional mathematical equation is independent of a constant 'k'.

9. One or more non-transitory machine-readable information storage mediums comprising one or more instructions, which when executed by one or more hardware processors, cause the one or more processors to carry out the method of claim 1.

**Patentansprüche**

1. Prozessorimplementiertes Verfahren (200) zur personalisierten Augenblinzelerkennung, wobei das Verfahren umfasst:

Erzeugen (202), durch einen oder mehrere Hardwareprozessoren, von personalisierten Augenblinzeldaten für ein Subjekt, die eine Mehrzahl von Bildern umfassen, die als Blinzeln oder Nicht-Blinzeln gekennzeichnet sind, wobei die Mehrzahl von Bildern durch eine Bilderfassungsvorrichtung zum Erfassen von Augenbereichsvideodaten des Subjekts für eine einmalige Kalibrierung eines personalisierten Blinzelschwellenwerts für das Subjekt erfasst wird, wobei ein Setup bereitgestellt wird, um einen Helligkeits- und Kontrastpegel durch das Subjekt anzupassen, und wobei das Setup das Subjekt anleitet, jedes der Mehrzahl von Bildern als Blinzeln oder Nicht-Blinzeln zu kennzeichnen;

Berechnen (204), durch den einen oder die mehreren Hardwareprozessoren, eines Augenseitenverhältnisses (EAR) für jedes der Mehrzahl von Bildern, um einen Satz von EAR-Werten zu erhalten;

Bestimmen (206), durch den einen oder die mehreren Hardwareprozessoren, des personalisierten Blinzelschwellenwerts durch Verarbeiten des Satzes von EAR-Werten, wobei der personalisierte Blinzelschwellenwert eines von i) einem ersten Blinzelschwellenwert, ii) einem zweiten Blinzelschwellenwert, iii) und einem Paar von Blinzelschwellenwerten umfasst, wobei der personalisierte Blinzelschwellenwert bestimmt wird durch Anwenden eines von:

a) einem ersten Schwellenwertansatz zum Berechnen des ersten Blinzelschwellenwerts basierend auf einem ersten Durchschnitt und einer ersten Standardabweichung von jedem des Satzes von EAR-Werten, die für jedes Bild in einem Blinzeln-gekennzeichneten Bildsatz aus der Mehrzahl von Bildern berechnet werden,

b) einem zweiten Schwellenwertansatz zum Berechnen des zweiten Blinzelschwellenwerts basierend auf einem basiskorrigierten Satz von EAR-Werten für die Mehrzahl von kommentierten Bildern unter Verwendung eines Minimums und eines zweiten Durchschnitts von jedem des basiskorrigierten Satzes von EAR-Werten für jedes Bild aus der Mehrzahl von Bildern, und

c) einem dritten Schwellenwertansatz zum Berechnen des Paars von Blinzelschwellenwerten basierend auf dem zweiten Durchschnitt von jedem des basiskorrigierten Satzes von EAR-Werten für jedes Bild aus dem Blinzeln-gekennzeichneten Bildsatz und einem Nicht-Blinzeln-gekennzeichneten Bildsatz aus der Mehrzahl von Bildern;

Erfassen einer Mehrzahl von Eingangstestbildern, durch den einen oder die mehreren Hardwareprozessoren, über ein vordefiniertes Zeitfenster; und

Verwenden (208), durch den einen oder die mehreren Hardwareprozessoren, des personalisierten Blinzelschwellenwerts zum Berechnen eines binären Entscheidungsvektors (D) für die Mehrzahl von Eingangstestbildern,

wobei der Wert eines Elements des binären Entscheidungsvektors (D), der für ein entsprechendes Eingangstestbild berechnet wird, auf '1' gesetzt wird, wenn ein EAR-Wert ($y_i$) des Eingangstestbilds unter dem ersten Blinzelschwellenwert liegt, wenn der personalisierte Blinzelschwellenwert unter Verwendung der ersten Schwellenwerttechnik berechnet wird,

wobei der Wert eines Elements des binären Entscheidungsvektors (D), der für ein entsprechendes Eingangstestbild berechnet wird, auf '1' gesetzt wird, wenn der basiskorrigierte EAR-Wert ($yc_i$) des Eingangstestbilds unter dem zweiten Blinzelschwellenwert liegt, wenn der personalisierte Blinzelschwellenwert unter Verwendung der zweiten Schwellenwerttechnik berechnet wird,

wobei der Wert eines Elements des binären Entscheidungsvektors (D), der für ein entsprechendes Eingangstestbild berechnet wird, auf '1' gesetzt wird, wenn die Differenz zwischen dem zweiten Durchschnitt für jedes Bild aus dem Blinzeln-gekennzeichneten Bildsatz und jedem des basiskorrigierten Satzes von EAR-Werten ($yc_i$) gleich oder unter der Differenz zwischen dem zweiten Durchschnitt für jedes Bild aus dem Nicht-Blinzeln-gekennzeichneten Bildsatz und jedem des basiskorrigierten Satzes von EAR-Werten liegt, wenn der personalisierte Blinzelschwellenwert unter Verwendung der dritten Schwellenwerttechnik berechnet wird, und

wobei die Mehrzahl von Eingangstestbildern als Augenblinzeln vorhergesagt wird, wenn eine definierte Anzahl von Elementen des binären Entscheidungsvektors mit dem Wert '1' für mindestens den vordefinierten Zeitraum detektiert wird.

2. Verfahren nach Anspruch 1, wobei der erste Blinzelschwellenwert mathematisch als $B_m + B_s$ ausgedrückt wird,

wobei $B_m$ der erste Durchschnitt des Satzes von EAR-Werten ist, die für jedes Bild aus dem Blinzeln-gekennzeichneten Bildsatz berechnet werden, und $B_s$ die erste Standardabweichung des Satzes von EAR-Werten ist, die für jedes Bild aus dem Blinzeln-gekennzeichneten Bildsatz berechnet werden.

3. Verfahren nach Anspruch 1, wobei der zweite Blinzelschwellenwert mathematisch als (t) ausgedrückt wird, wobei $t = xc_m - k \times (xc_m - xc_{min})$, wobei $xc_m$ der zweite Durchschnitt von jedem des basiskorrigierten Satzes von EAR-Werten für jedes Bild aus der Mehrzahl von Bildern ist, und $xc_{min}$ das Minimum von jedem des basiskorrigierten Satzes von EAR-Werten für jedes Bild aus der Mehrzahl von Bildern ist, und wobei k eine empirisch abgeleitete Konstante ist.

4. Verfahren nach Anspruch 1, wobei das Paar von Schwellenwerten ein Parameter einer bedingten mathematischen Gleichung ist, die als $|xc_{Mb} - yc_i| \leq |xc_{Mnb} - yc_i|$ ausgedrückt wird, wobei $xc_{Mb}$ der zweite Durchschnitt von jedem des basiskorrigierten Satzes von EAR-Werten für jedes Bild aus dem Blinzeln-gekennzeichneten Bildsatz ist, und $xc_{Mnb}$ der zweite Durchschnitt von jedem des basiskorrigierten Satzes von EAR-Werten für jedes Bild aus dem Nicht-Blinzeln-gekennzeichneten Bildsatz ist, und wobei die bedingte mathematische Gleichung unabhängig von einer Konstante 'k' ist.

5. System (100) zur personalisierten Augenblinzelerkennung, wobei das System (100) umfasst:

einen Speicher (102), der Anweisungen speichert;
eine oder mehrere Eingabe/Ausgabe(E/A)-Schnittstellen (106); und
einen oder mehrere Hardwareprozessoren (104), die mit dem Speicher (102) über die eine oder die mehreren E/A-Schnittstellen (106) gekoppelt sind, wobei der eine oder die mehreren Hardwareprozessoren (104) durch die Anweisungen konfiguriert sind zum:

Erzeugen von personalisierten Augenblinzeldaten für ein Subjekt von Interesse, die eine Mehrzahl von Bildern umfassen, die als Blinzeln oder Nicht-Blinzeln gekennzeichnet sind, wobei die Mehrzahl von Bildern durch eine Bilderfassungsvorrichtung zum Erfassen von Augenbereichsvideodaten des Subjekts für eine einmalige Kalibrierung eines personalisierten Blinzelschwellenwerts für das Subjekt erfasst wird, wobei ein Setup bereitgestellt wird, um einen Helligkeits- und Kontrastpegel durch das Subjekt anzupassen, und wobei das Setup das Subjekt anleitet, jedes der Mehrzahl von Bildern als Blinzeln oder Nicht-Blinzeln zu kennzeichnen;
Berechnen eines Augenseitenverhältnisses (EAR) für jedes der Mehrzahl von Bildern, um einen Satz von EAR-Werten zu erhalten;
Bestimmen des personalisierten Blinzelschwellenwerts durch Verarbeiten des Satzes von EAR-Werten, wobei der personalisierte Blinzelschwellenwert eines von i) einem ersten Blinzelschwellenwert, ii) einem zweiten Blinzelschwellenwert, iii) und einem Paar von Blinzelschwellenwerten umfasst, wobei der personalisierte Blinzelschwellenwert bestimmt wird durch Anwenden eines von:

a) einem ersten Schwellenwertansatz zum Berechnen des ersten Blinzelschwellenwerts basierend auf einem ersten Durchschnitt und einer ersten Standardabweichung von jedem des Satzes von EAR-Werten, die für jedes Bild in einem Blinzeln-gekennzeichneten Bildsatz aus der Mehrzahl von Bildern berechnet werden,
b) einem zweiten Schwellenwertansatz zum Berechnen des zweiten Blinzelschwellenwerts basierend auf einem basiskorrigierten Satz von EAR-Werten für die Mehrzahl von kommentierten Bildern unter Verwendung eines Minimums und eines zweiten Durchschnitts von jedem des basiskorrigierten Satzes von EAR-Werten für jedes Bild aus der Mehrzahl von Bildern, und
c) einem dritten Schwellenwertansatz zum Berechnen des Paars von Blinzelschwellenwerten basierend auf dem zweiten Durchschnitt von jedem des basiskorrigierten Satzes von EAR-Werten für jedes Bild aus dem Blinzeln-gekennzeichneten Bildsatz und einem Nicht-Blinzeln-gekennzeichneten Bildsatz aus der Mehrzahl von Bildern;

Erfassen einer Mehrzahl von Eingangstestbildern über ein vordefiniertes Zeitfenster;
Verwenden des personalisierten Blinzelschwellenwerts zum Berechnen eines binären Entscheidungsvektors (D) für die Mehrzahl von Eingangstestbildern,
wobei der Wert eines Elements des binären Entscheidungsvektors (D), der für ein entsprechendes Eingangstestbild berechnet wird, auf '1' gesetzt wird, wenn ein EAR-Wert ($y_i$) des Eingangstestbilds unter dem ersten Blinzelschwellenwert liegt, wenn der personalisierte Blinzelschwellenwert unter Verwendung der ersten Schwellenwerttechnik berechnet wird,

wobei der Wert eines Elements des binären Entscheidungsvektors (D), der für ein entsprechendes Eingangstestbild berechnet wird, auf '1' gesetzt wird, wenn der basiskorrigierte EAR-Wert ($yc_i$) des Eingangstestbilds unter dem zweiten Blinzelschwellenwert liegt, wenn der personalisierte Blinzelschwellenwert unter Verwendung der zweiten Schwellenwerttechnik berechnet wird,

wobei der Wert eines Elements des binären Entscheidungsvektors (D), der für ein entsprechendes Eingangstestbild berechnet wird, auf '1' gesetzt wird, wenn die Differenz zwischen dem zweiten Durchschnitt für jedes Bild aus dem Blinzeln-gekennzeichneten Bildsatz und jedem des basiskorrigierten Satzes von EAR-Werten ($yc_i$) gleich oder unter der Differenz zwischen dem zweiten Durchschnitt für jedes Bild aus dem Nicht-Blinzeln-gekennzeichneten Bildsatz und jedem des basiskorrigierten Satzes von EAR-Werten liegt, wenn der personalisierte Blinzelschwellenwert unter Verwendung der dritten Schwellenwerttechnik berechnet wird, und

wobei die Mehrzahl von Eingangstestbildern als Augenblinzeln vorhergesagt wird, wenn eine definierte Anzahl von Elementen des binären Entscheidungsvektors mit dem Wert '1' für mindestens den vordefinierten Zeitraum detektiert wird.

6. System nach Anspruch 5, wobei der erste Blinzelschwellenwert mathematisch als $B_m + B_s$ ausgedrückt wird, wobei $B_m$ der erste Durchschnitt des Satzes von EAR-Werten ist, die für jedes Bild aus dem Blinzeln-gekennzeichneten Bildsatz berechnet werden, und $B_s$ die erste Standardabweichung des Satzes von EAR-Werten ist, die für jedes Bild aus dem Blinzeln-gekennzeichneten Bildsatz berechnet werden.

7. System nach Anspruch 5, wobei der zweite Blinzelschwellenwert mathematisch als (t) ausgedrückt wird, wobei $t = xc_m - k \times (xc_m - xc_{min})$, wobei $xc_m$ der zweite Durchschnitt von jedem des basiskorrigierten Satzes von EAR-Werten für jedes Bild aus der Mehrzahl von Bildern ist, und $xc_{min}$ das Minimum von jedem des basiskorrigierten Satzes von EAR-Werten für jedes Bild aus der Mehrzahl von Bildern ist, und wobei k eine empirisch abgeleitete Konstante ist.

8. System nach Anspruch 5, wobei das Paar von Schwellenwerten Parameter einer bedingten mathematischen Gleichung sind, die als $|xc_{Mb} - yc_i| \leq |xc_{Mnb} - yc_i|$ ausgedrückt wird, wobei $xc_{Mb}$ der zweite Durchschnitt von jedem des basiskorrigierten Satzes von EAR-Werten für jedes Bild aus dem Blinzeln-gekennzeichneten Bildsatz ist, und $xc_{Mnb}$ der zweite Durchschnitt von jedem des basiskorrigierten Satzes von EAR-Werten für jedes Bild aus dem Nicht-Blinzeln-gekennzeichneten Bildsatz ist, und wobei die bedingte mathematische Gleichung unabhängig von einer Konstante 'k' ist.

9. Ein oder mehrere nichtflüchtige maschinenlesbare Informationsspeichermedien, die eine oder mehrere Anweisungen umfassen, die, wenn sie durch einen oder mehrere Hardwareprozessoren ausgeführt werden, den einen oder die mehreren Prozessoren veranlassen, das Verfahren nach Anspruch 1 auszuführen.

## Revendications

1. Procédé mis en oeuvre par processeur (200) pour une détection personnalisée de battements de paupières, le procédé comprend les étapes ci-dessous consistant à :

créer (202), par le biais d'un ou plusieurs processeurs matériels, des données personnalisées de battements de paupières pour un sujet comprenant une pluralité d'images étiquetées comme étant « avec battements » ou « sans battements », dans laquelle la pluralité d'images est capturée par un dispositif de capture d'images pour capturer des données vidéo de région des yeux du sujet pour un étalonnage unique d'un seuil de battement personnalisé pour le sujet, dans lequel une configuration est fournie pour ajuster des niveaux de luminosité et de contraste par le sujet, et dans lequel la configuration guide le sujet pour étiqueter chacune de la pluralité d'images comme étant avec battements ou sans battements ;

calculer (204), par le biais dudit un ou desdits plusieurs processeurs matériels, un rapport d'aspect d'oeil (EAR) pour chacune de la pluralité d'images, afin d'obtenir un ensemble de valeurs de rapport EAR ;

déterminer (206), par le biais dudit un ou desdits plusieurs processeurs matériels, le seuil de battement personnalisé, en traitant l'ensemble de valeurs de rapport EAR, dans lequel le seuil de battement personnalisé comprend l'une parmi i) une première valeur de seuil de battement, ii) une deuxième valeur de seuil de battement, iii) et une paire de valeurs de seuil de battement, dans lequel le seuil de battement personnalisé est déterminé en appliquant l'une parmi :

a) une première approche de seuillage pour calculer la première valeur de seuil de battement sur la base d'une première moyenne et d'un premier écart type de chaque valeur de l'ensemble de valeurs de rapport EAR calculées pour chaque image d'un ensemble d'images étiquetées comme étant avec battements parmi la pluralité d'images ;

b) une deuxième approche de seuillage pour calculer la deuxième valeur de seuil de battement sur la base d'un ensemble de valeurs de rapport EAR à ligne de base corrigée pour la pluralité d'images annotées, en utilisant un minimum et une deuxième moyenne de chaque valeur de l'ensemble de valeurs de rapport EAR à ligne de base corrigée pour chaque image parmi la pluralité d'images ; et

c) une troisième approche de seuillage pour calculer la paire de valeurs de seuil de battement sur la base de la deuxième moyenne de chaque valeur de l'ensemble de valeurs de rapport EAR à ligne de base corrigée pour chaque image de l'ensemble d'images étiquetées comme étant avec battements et d'un ensemble d'images étiquetées comme étant sans battements parmi la pluralité d'images ;

capturer une pluralité d'images de test d'entrée, par le biais dudit un ou desdits plusieurs processeurs matériels, au cours d'une fenêtre temporelle prédéfinie ; et

utiliser (208), par le biais dudit un ou desdits plusieurs processeurs matériels, le seuil de battement personnalisé, pour calculer un vecteur de décision binaire (D) pour la pluralité d'images de test d'entrée ;

dans lequel la valeur d'un élément du vecteur de décision binaire (D) calculé pour une image de test d'entrée correspondante est définie sur « 1 » si une valeur de rapport EAR ($y_i$) de l'image de test d'entrée est inférieure à la première valeur de seuil de battement lorsque la valeur de seuil de battement personnalisée est calculée à l'aide de la première technique de seuillage ;

dans lequel la valeur d'un élément du vecteur de décision binaire (D) calculé pour une image de test d'entrée correspondante est définie sur « 1 » si la valeur de rapport EAR à ligne de base corrigée ($yc_i$) de l'image de test d'entrée est inférieure à la deuxième valeur de seuil de battement lorsque la valeur de seuil de battement personnalisée est calculée à l'aide de la deuxième technique de seuillage ;

dans lequel la valeur d'un élément du vecteur de décision binaire (D) calculé pour une image de test d'entrée correspondante est définie sur « 1 » si la différence entre la deuxième moyenne pour chaque image de l'ensemble d'images étiquetées comme étant avec battements et chaque valeur de l'ensemble de valeurs de rapport EAR à ligne de base corrigée ($yc_i$) est égale ou inférieure à la différence entre la deuxième moyenne pour chaque image de l'ensemble d'images étiquetées comme étant sans battements et chaque valeur de l'ensemble de valeurs de rapport EAR à ligne de base corrigée, lorsque la valeur de seuil de battement personnalisée est calculée à l'aide de la troisième technique de seuillage ; et

dans lequel la pluralité d'images de test d'entrée est prédite comme correspondant à un battement de paupière si un nombre défini d'éléments du vecteur de décision binaire présentant la valeur « 1 » sont détectés pendant au moins la période de temps prédéfinie.

2. Procédé selon la revendication 1, dans lequel la première valeur de seuil de battement est exprimée mathématiquement en tant que $B_m + B_s$, dans lequel « $B_m$ » est la première moyenne de l'ensemble de valeurs de rapport EAR calculées pour chaque image parmi l'ensemble d'images étiquetées comme étant avec battements et « $B_s$ » est le premier écart type de l'ensemble de valeurs de rapport EAR calculées pour chaque image parmi l'ensemble d'images étiquetées comme étant avec battements.

3. Procédé selon la revendication 1, dans lequel la deuxième valeur de seuil de battement est exprimée mathématiquement en tant que (t), dans lequel $t = xc_m - k \times (xc_m - xc_{min})$, dans lequel « $xc_m$ » est la deuxième moyenne de chaque valeur de l'ensemble de valeurs de rapport EAR à ligne de base corrigée pour chaque image parmi la pluralité d'images, et « $xc_{min}$ » est le minimum de chaque valeur de l'ensemble de valeurs de rapport EAR à ligne de base corrigée pour chaque image parmi la pluralité d'images, et dans lequel « k » est une constante dérivée de manière empirique.

4. Procédé selon la revendication 1, dans lequel la paire de valeurs de seuil est un paramètre d'une équation mathématique conditionnelle exprimée en tant que $|xc_{Mb} - yc_i| \leq |xc_{Mnb} - yc_i|$, dans laquelle « $xc_{Mb}$ » est la deuxième moyenne de chaque valeur de l'ensemble de valeurs de rapport EAR à ligne de base corrigée pour chaque image de l'ensemble d'images étiquetées comme étant avec battements, et « $xc_{Mnb}$ » est la deuxième moyenne de chaque valeur de l'ensemble de valeurs de rapport EAR à ligne de base corrigée pour chaque image de l'ensemble d'images étiquetées comme étant sans battements, et dans laquelle l'équation mathématique conditionnelle est indépendante d'une constante « k ».

5. Système (100) pour une détection personnalisée de battements de paupières, le système (100) comprenant :

une mémoire (102) stockant des instructions ;

une ou plusieurs interfaces d'entrée/sortie (I/O) (106) ; et

un ou plusieurs processeurs matériels (104) couplés à la mémoire (102) par l'intermédiaire de ladite une ou desdites plusieurs interfaces I/O (106), dans lesquels ledit un ou lesdits plusieurs processeurs matériels (104) sont configurés, par les instructions, de manière à :

créer des données personnalisées de battements de paupières pour un sujet d'intérêt comprenant une pluralité d'images étiquetées comme étant « avec battements » ou « sans battements », dans laquelle la pluralité d'images est capturée par un dispositif de capture d'images pour capturer des données vidéo de région des yeux du sujet pour un étalonnage unique d'un seuil de battement personnalisé pour le sujet, dans lequel une configuration est fournie pour ajuster des niveaux de luminosité et de contraste par le sujet, et dans lequel la configuration guide le sujet pour étiqueter chacune de la pluralité d'images comme étant avec battements ou sans battements ;

calculer un rapport d'aspect d'oeil (EAR) pour chacune de la pluralité d'images, afin d'obtenir un ensemble de valeurs de rapport EAR ;

déterminer le seuil de battement personnalisé, en traitant l'ensemble de valeurs de rapport EAR, dans lequel le seuil de battement personnalisé comprend l'une parmi i) une première valeur de seuil de battement, ii) une deuxième valeur de seuil de battement, iii) et une paire de valeurs de seuil de battement, dans lequel le seuil de battement personnalisé est déterminé en appliquant l'une parmi :

a) une première approche de seuillage pour calculer la première valeur de seuil de battement sur la base d'une première moyenne et d'un premier écart type de chaque valeur de l'ensemble de valeurs de rapport EAR calculées pour chaque image d'un ensemble d'images étiquetées comme étant avec battements parmi la pluralité d'images ;

b) une deuxième approche de seuillage pour calculer la deuxième valeur de seuil de battement sur la base d'un ensemble de valeurs de rapport EAR à ligne de base corrigée pour la pluralité d'images annotées, en utilisant un minimum et une deuxième moyenne de chaque valeur de l'ensemble de valeurs de rapport EAR à ligne de base corrigée pour chaque image parmi la pluralité d'images ; et

c) une troisième approche de seuillage pour calculer la paire de valeurs de seuil de battement sur la base de la deuxième moyenne de chaque valeur de l'ensemble de valeurs de rapport EAR à ligne de base corrigée pour chaque image de l'ensemble d'images étiquetées comme étant avec battements et d'un ensemble d'images étiquetées comme étant sans battements parmi la pluralité d'images ;

capturer une pluralité d'images de test d'entrée au cours d'une fenêtre temporelle prédéfinie ; et

utiliser le seuil de battement personnalisé pour calculer un vecteur de décision binaire (D) pour la pluralité d'images de test d'entrée ;

dans lequel la valeur d'un élément du vecteur de décision binaire (D) calculé pour une image de test d'entrée correspondante est définie sur « 1 » si une valeur de rapport EAR ($y_i$) de l'image de test d'entrée est inférieure à la première valeur de seuil de battement lorsque la valeur de seuil de battement personnalisée est calculée à l'aide de la première technique de seuillage ;

dans lequel la valeur d'un élément du vecteur de décision binaire (D) calculé pour une image de test d'entrée correspondante est définie sur « 1 » si la valeur de rapport EAR à ligne de base corrigée ($yc_i$) de l'image de test d'entrée est inférieure à la deuxième valeur de seuil de battement lorsque la valeur de seuil de battement personnalisée est calculée à l'aide de la deuxième technique de seuillage ;

dans lequel la valeur d'un élément du vecteur de décision binaire (D) calculé pour une image de test d'entrée correspondante est définie sur « 1 » si la différence entre la deuxième moyenne pour chaque image de l'ensemble d'images étiquetées comme étant avec battements et chaque valeur de l'ensemble de valeurs de rapport EAR à ligne de base corrigée ($yc_i$) est égale ou inférieure à la différence entre la deuxième moyenne pour chaque image de l'ensemble d'images étiquetées comme étant sans battements et chaque valeur de l'ensemble de valeurs de rapport EAR à ligne de base corrigée, lorsque la valeur de seuil de battement personnalisée est calculée à l'aide de la troisième technique de seuillage ; et

dans lequel la pluralité d'images de test d'entrée est prédite comme correspondant à un battement de paupière si un nombre défini d'éléments du vecteur de décision binaire présentant la valeur « 1 » sont détectés pendant au moins la période de temps prédéfinie.

**6.** Système selon la revendication 5, dans lequel la première valeur de seuil de battement est exprimée mathématiquement en tant que $B_m + B_s$, dans lequel « $B_m$ » est la première moyenne de l'ensemble de valeurs de rapport EAR calculées pour chaque image parmi l'ensemble d'images étiquetées comme étant avec battements et « $B_s$ »

est le premier écart type de l'ensemble de valeurs de rapport EAR calculées pour chaque image parmi l'ensemble d'images étiquetées comme étant avec battements.

7. Système selon la revendication 5, dans lequel la deuxième valeur de seuil de battement est exprimée mathématiquement en tant que (t), dans lequel t = $xc_m$ - k × ($xc_m$ - $xc_{min}$), dans lequel « $xc_m$ » est la deuxième moyenne de chaque valeur de l'ensemble de valeurs de rapport EAR à ligne de base corrigée pour chaque image parmi la pluralité d'images, et « $xc_{min}$ » est le minimum de chaque valeur de l'ensemble de valeurs de rapport EAR à ligne de base corrigée pour chaque image parmi la pluralité d'images, et dans lequel « k » est une constante dérivée de manière empirique.

8. Système selon la revendication 5, dans lequel la paire de valeurs de seuil est un paramètre d'une équation mathématique conditionnelle exprimée en tant que $|xc_{Mb} - yc_i| \leq |xc_{Mnb} - yc_i|$, dans laquelle « $xc_{Mb}$ » est la deuxième moyenne de chaque valeur de l'ensemble de valeurs de rapport EAR à ligne de base corrigée pour chaque image de l'ensemble d'images étiquetées comme étant avec battements, et « $xc_{Mnb}$ » est la deuxième moyenne de chaque valeur de l'ensemble de valeurs de rapport EAR à ligne de base corrigée pour chaque image de l'ensemble d'images étiquetées comme étant sans battements, et dans laquelle l'équation mathématique conditionnelle est indépendante d'une constante « k ».

9. Un ou plusieurs supports de stockage non transitoires d'informations lisibles par machine comprenant une ou plusieurs instructions qui, lorsqu'elles sont exécutées par un ou plusieurs processeurs matériels, amènent ledit un ou lesdits plusieurs processeurs à mettre en oeuvre le procédé selon la revendication 1.

System**100**

Processor(s) **104**

I/O Interface(s) **106**

Memory **102**

Database **108**

FIG. 1

200

create personalized eye blink data for a subject comprising a plurality of images tagged as blink or no-blink, wherein the plurality of images are captured by an image capturing device for one time calibration of a personalized blink threshold for the subject ⌐ 202

compute Eye Aspect Ratio (EAR) for each of the plurality of images to obtain a set of EAR values ⌐ 204

determine the personalized blink threshold by processing the set of EAR values, wherein the personalized blink threshold comprising one of i) a first blink threshold value, ii) a second blink threshold value, iii) and a pair of blink threshold values, wherein the personalized blink threshold is determined by applying one of: a first thresholding approach, a second thresholding approach, and a third thresholding approach ⌐ 206

utilize the personalized blink threshold to compute a binary decision vector (D) for a plurality of input test images captured over a predefined time window, wherein the plurality of input test images are predicted as eye blink if number of elements of the binary decision vector having value '1' are detected for at least the predefined time period ⌐ 208

**FIG. 2**

FIG. 3

FIG. 4A

Please gaze at '+' symbol and blink only when you hear beep sound

+

**FIG. 4B**

FIG. 4C

FIG. 5

FIG. 6

**FIG. 7**

**EP 4 105 827 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202121026457

**Non-patent literature cited in the description**

- **KUMAR ASHISH et al.** *Driver drowsiness monitoring system using visual behaviour and machine learning*

- **HUDA et al.** Mobile-based driver sleepiness detection using facial landmarks and analysis of EAR values. *SOA1*
- **MALLIKARJUNA.** Liveness detection based on human eye blinking for photo attacks. *SOA2*